(19) Europäisches Patentamt
European Patent Office
Office européen des brevets

(11) Numéro de publication : **0 508 901 A2**

(12) # DEMANDE DE BREVET EUROPEEN

(21) Numéro de dépôt : **92401003.6**

(22) Date de dépôt : **09.04.92**

(51) Int. Cl.[5] : **C07D 277/30,** A01N 43/78, C07D 277/32, C07D 277/34, C07D 277/36, C07C 69/716

(30) Priorité : **10.04.91 FR 9104340**

(43) Date de publication de la demande : **14.10.92 Bulletin 92/42**

(84) Etats contractants désignés : **AT BE CH DE DK ES FR GB GR IT LI LU NL PT SE**

(71) Demandeur : **ROUSSEL-UCLAF**
**35, Boulevard des Invalides**
**F-75007 Paris (FR)**

(72) Inventeur : **Brayer, Jean-Louis**
**42, rue Jules Dubrulle**
**F-60440 Nanteuil le Haudoin (FR)**
Inventeur : **Demoute, Jean-Pierre**
**65, Avenue Foch**
**F-93360 Neuilly Plaisance (FR)**
Inventeur : **Mourioux, Gilles**
**12, Allée des Micocouliers**
**F-13420 Gemenos (FR)**

(74) Mandataire : **Vieillefosse, Jean-Claude et al**
**Roussel-Uclaf 111, route de Noisy B.P. 9**
**F-93230 Romainville (FR)**

(54) **Dérivés de l'acide alpha-méthylène 4-[(phénoxy)méthyl] 5-thiazolacétique, leur procédé de préparation et les intermédiaires de ce procédé, leur application à titre de fongicides et les compositions les renfermant.**

(57) L'invention a pour objet les produits de formule (I) :

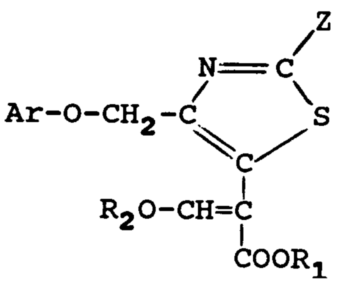

(I)

dans laquelle Ar représente un radical phényle non substitué ou substitué, Z représente un atome d'hydrogène, un atome de chlore, un radical trifluorométhyle, un radical alkyle, un radical alkyloxy ou un radical alkylthio ($C_{1-6}$), $R_1$ et $R_2$ reréséntent un radical alkyle ($C_{1-6}$) et la double liaison exocylique est en configuration (Z) ou (E), leur procédé de préparation et les intermédiaires de ce procédé, leur application à titre de fongicides et les compositions les renfermant.

EP 0 508 901 A2

La présente invention concerne de nouveaux dérivés de l'acide alpha-méthylène 4-[(phénoxy) méthyl] 5-thiazolacétique, leur procédé de préparation et les intermédiaires de ce procédé, leur application à titre de fongicides et les compositions les renfermant.

L'invention a pour objet les produits de formule (I) :

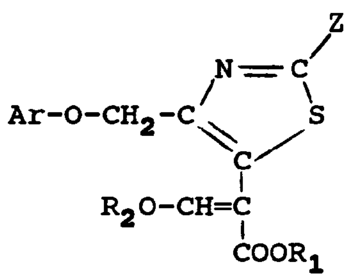

(I)

dans laquelle Ar représente un radical phényle non substitué ou substitué par un ou plusieurs radicaux identiques ou différents choisis parmi les atomes d'halogène, le radical méthylènedioxy, le radical phénoxy, le radical phényle, le radical trifluorométhyle, les radicaux alkyle linéaires ou ramifiés renfermant de un à six atomes de carbone, les radicaux alkyloxy linéaires ou ramifiés renfermant de un à six atomes de carbone ou les radicaux alkylthio linéaires ou ramifiés renfermant de un à six atomes de carbone, Z représente un atome d'hydrogène, un atome de chlore, un radical trifluorométhyle, un radical alkyle linéaire ou ramifié renfermant de un à six atomes de carbone, un radical alkyloxy linéaire ou ramifié renfermant de un à six atomes de carbone, un radical alkylthio linéaire ou ramifié renfermant de un à six atomes de carbone, $R_1$ et $R_2$ identiques ou différents représentent indépendamment l'un de l'autre, un radical alkyle linéaire ou ramifié renfermant de un à six atomes de carbone et la double liaison exocyclique est en configuration (Z) ou en configuration (E).

Par radical alkyle linéaire ou ramifié renfermant de un à six atomes de carbone, on entend les radicaux méthyle, éthyle, propyle, butyle, pentyle ou hexyle et notamment le radical méthyle, éthyle, propyle, isopropyle, butyle, isobutyle, (R)-sec-butyle, (S)-sec-butyle ou tert-butyle.

Par radical alkyloxy linéaire ou ramifié renfermant de un à six atomes de carbone, on entend un radical alkyloxy dont la composante alkyle est linéaire ou ramifiée et renferme de un à six atomes de carbone et notamment le radical méthoxy, éthoxy, propoxy, isopropoxy, butoxy, isobutoxy, (R)-sec-butoxy, (S)-sec-butoxy ou tert-butoxy.

Par radical alkylthio linéaire ou ramifié renfermant de un à six atomes de carbone, on entend un radical alkylthio dont la composante alkyle est linéaire ou ramifiée et renferme de un à six atomes de carbone et notamment le radical méthylthio, éthylthio, propylthio, isopropylthio, butylthio, isobutylthio, (R)-sec-butylthio, (S)-sec-butylthio ou tert-butylthio.

Lorsque Ar représente un radical phényle substitué par un ou plusieurs atomes d'halogène, il représente notamment l'un des radicaux 2-bromo phényle, 3-bromo phényle, 4-bromo phényle, 2-chloro phényle, 3-chloro phényle, 4-chloro phényle, 2,6-dichloro phényle, 2,3,5-trichloro phényle, 2-fluoro phényle, 3-fluoro phényle, 4-fluoro phényle, 2,3-difluoro phényle, 2,4-difluoro phényle, 2,5-difluoro phényle, 2,6-difluoro phényle, 3,4-difluoro phényle, 3,5-difluoro phényle, 2,3,5,6-tétrafluoro phényle, 2,3,4,5,6-pentafluoro phényle, 2-chloro 6-fluoro phényle ou 3-chloro 4-fluoro phényle.

Lorsque Ar représente un radical phényle substitué par un ou plusieurs radicaux alkyle, il représente notamment un des radicaux ortho, méta ou para-tolyle, 2,4-diméthyl phényle, ou mésityle.

Lorsque Ar représente un radical phényle substitué par un ou plusieurs radicaux alkyloxy, il représente notamment un des radicaux 2-méthoxy phényle, 3-méthoxy phényle, 4-méthoxy phényle, 4-éthoxy phényle, 4-butoxy phényle, 2,4-diméthoxy phényle ou 3,4,5-triméthoxy phényle.

Lorsque Ar représente un radical phényle substitué par un ou plusieurs radicaux alkylthio, il représente notamment un radical 4-méthylthio phényle.

Lorsque Ar représente un radical phényle substitué par plusieurs radicaux différents, il représente notamment un des radicaux 5-bromo 2-méthoxy phényle, 3-bromo 4,5-diméthoxy phényle, 6-bromo 3,4-diméthoxy phényle ou 4-méthoxy 3-méthyl phényle.

L'invention a particulièrement pour objet les produits de formule (I) telle que définie précédemment dans laquelle $R_1$ et $R_2$ représentent un radical méthyle, ceux dans laquelle Z représente un atome d'hydrogène, un atome de chlore ou un des radicaux choisis parmi les radicaux méthyle, éthyle, propyle, isopropyle, méthoxy, éthoxy et méthylthio et spécialement ceux dont les noms suivent :

– alpha-[(Z)-méthoxy méthylène] 4-(phénoxyméthyl) 5-thiazolacétate de méthyle,

– 2-chloro alpha-[(Z)-méthoxy méthylène] 4-(phénoxyméthyl) 5-thiazolacétate de méthyle.

L'invention a également pour objet un procédé de préparation des produits de formule (I) telle que définie précédemment, caractérisé en ce qu'un ester de l'acide 5-halo 4-oxo pentanoïque de formule (II) :

$X-CH_2-CO-CH_2-CH_2-COOR_1$ (II)

dans laquelle X représente un atome de chlore ou de brome et $R_1$ a la signification indiquée ci-dessus, est traité en présence d'une base avec un phénol de formule (III) :

Ar-OH (III)

dans laquelle Ar a la signification indiquée ci-dessus, pour obtenir l'ester de l'acide 5-(aryloxy) 4-oxo pentanoïque de formule (IV) :

$Ar-O-CH_2-CO-CH_2-CH_2-COOR_1$ (IV)

ester de formule (IV), que l'on transforme par action d'un trialkyl chloro silane de formule (V) :

$R_3R_4R_5SiCl$ (V)

dans laquelle $R_3$, $R_4$ et $R_5$ identiques ou différents, représentent un radical alkyle linéaire ou ramifié renfermant de un à quatre atomes de carbone, en un ether d'énol silylé de formule (VI) :

$Ar-O-CH_2-C(OSiR_3R_4R_5)=CH-CH_2-COOR_1$ (VI)

produit de formule (VI) qui, par bromation permet d'obtenir le dérivé 3-bromo de l'ester de l'acide 5-(aryloxy) 4-oxo pentanoïque de formule (VII) :

$Ar-O-CH_2-CO-CHBr-CH_2-COOR_1$ (VII)

que l'on fait réagir :

soit avec le produit de formule (VIII) :

$Z_1-CS-NH_2$ (VIII)

dans laquelle $Z_1$ représente un atome d'hydrogène ou un radical alkyle linéaire ou ramifié, renfermant de un à six atomes de carbone, pour former un produit de formule $(IX)_1$ :

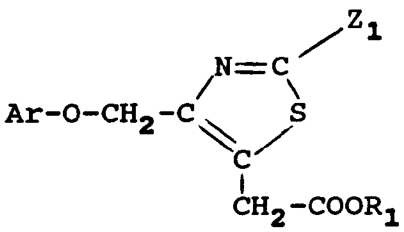

$(IX)_1$

soit avec un produit de formule (X) :

$Alc_1-O-CS-NH_2$ (X)

dans laquelle $Alc_1$ représente un radical alkyle renfermant de 1 à 4 atomes de carbone, pour former le dérivé de la 2-thiazolinone de formule (XI) :

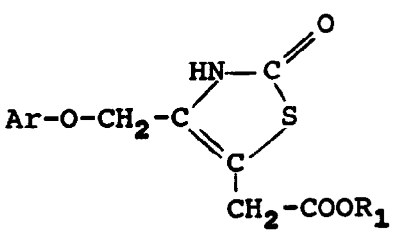

(XI)

produit de formule (XI) que l'on traite avec un réactif de chloration des fonctions carbonyle pour obtenir le produit de formule $(IX)_2$ :

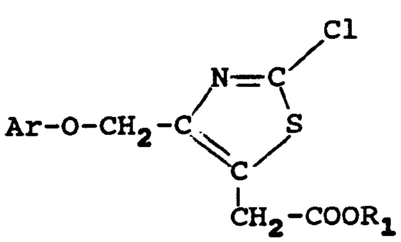

$(IX)_2$

produit de formule (XI) qui si désiré, est mis à réagir avec une base de formule (XII) :

$Z_3^- M^+$ (XII)

dans laquelle $Z_3^-$ représente un anion alcoolate et $M^+$ un cation ammonium ou un cation de métal alcalin, pour conduire au produit de formule $(IX)_3$ :

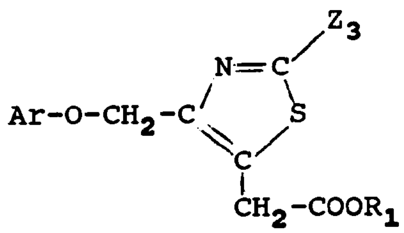

$(IX)_3$

soit avec un dithiocarbamate de formule (XIII) :

$NH_2\text{-CS-S}^- M^+$ (XIII)

pour obtenir le dérivé de la 2-thiazolinethione de formule (XIV) :

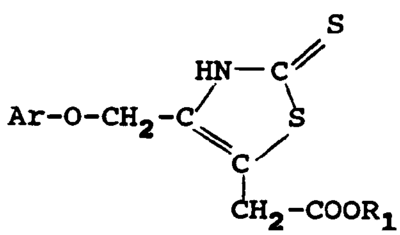

(XIV)

produits de formules $(IX)_1$, $(IX)_2$, $(IX)_3$ ou (XIV), que l'on condense en présence d'une base avec un acétal du diméthyl formamide de formule (XV) :

$Me_2N\text{-CH}(OR_7)_2$ (XV)

dans laquelle $R^7$ représente un radical alkyle linéaire ou ramifié renfermant de un à six atomes de carbone, pour obtenir respectivement les produits de formules $(XVI)_1$, $(XVI)_2$, $(XVI)_3$, et $(XVI)_4$ :

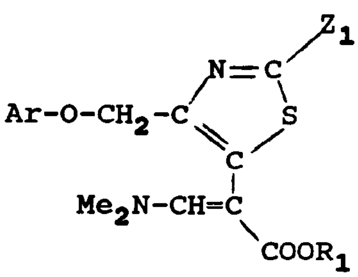

$(XVI)_1$,

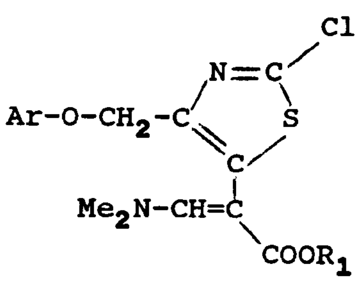

$(XVI)_2$,

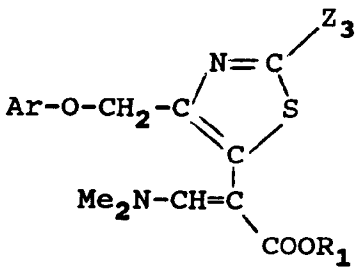

(XVI)$_3$ et

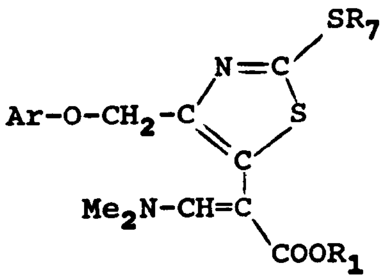

(XVI)$_4$

produits de formules (XVI)$_1$, (XVI)$_2$, (XVI)$_3$ et (XVI)$_4$ que l'on peut représenter sous la seule formule (XVI) :

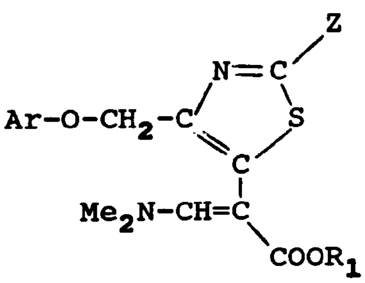

(XVI)

dans laquelle Z, Ar et $R_1$ a la signification indiquée ci-dessus, et produit de formule (XVI) qui, par hydrolyse est transformé en produit de formule (XVII) :

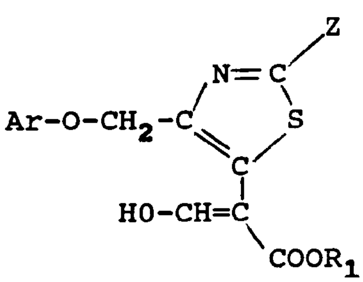

(XVII)

produit de formule (XVII) que l'on éthérifie en produit de formule (I).

Dans un mode préféré d'éxécution du procédé défini ci-dessus, le trialkyl chlorosilane utilisé pour la synthèse de l'éther d'énol silylé de formule (VI) est par exemple le chloro triméthyl silane ou le tert-butyl chloro diméthyl silane ; la réaction est effectuée en présence d'une base azotée telle que le 1,5-diaza 5-bicyclo[5.4.0]undécène (DBU) ou le 1,5-diaza 5-bicyclo[4.3.0]nonène (DBN) ; le produit de formule (VI) est bromé sans purification préalable par un agent de bromation classique tel que le brome ou le N-bromo succinimide (NBS) ;
la réaction de cyclisation en produit de formule (IX)$_1$ se fait en milieu alcoolique, par exemple dans le méthanol ou l'éthanol ;
la préparation des produits de formule (XI) se fait par exemple par action du thiocarbamate d'éthyle sur le produit de formule (VII) dans le méthanol ou l'éthanol ;
la chloration en produit de formule (IX)$_2$ se fait par exemple par action par le chlorure de phosphoryle en présence d'une base azotée telle que la 2,6-lutidine ; l'alcoolate de formule (XII) utilisé pour l'obtention du produit de formule $(IX)_3$ est par exemple le méthylate de sodium ou l'éthylate de sodium ; le dithiocarbamate utilisé pour la préparation du produit de formule (XIV) est le dithiocarbamate d'ammonium ;
l'éthérification du produit de formule (XVII) est effectuée au moyen d'un halogénure d'alkyle, par exemple l'iodure de méthyle ;
la préparation des produits de formule (I) à partir des produits de formule (XVI) peut être effectuée sans isoler l'intermédiaire de formule (XVII) ; la préparation des produits de formules $(IX)_1$, (XI) et (XIV) peut être effectuée sans isoler l'ester de formule (VII).

Les produits de départ de formule (II) peuvent être préparés à partir de l'acide 4-oxo pentanoïque (ou acide lévulinique) qui est un produit commercial, par estérification de cet acide suivie d'une halogénation en position alpha de la fonction carbonyle comme celà est décrit plus loin dans la préparation du 5-bromo 4-oxo pentanoate de méthyle ; certains produits de formule (II) sont connus et leur préparation est décrite dans : Pichat et coll. ; Bulletin de la Société Chimique de France (1956) page 1750 ; Rappe, Ark. Kemi Volume 14, (1959) pages 467 à 469 ; Dannenberg et Laüfer, Chemisches Bericht volume 89, (1956) pages 2242 à 2252 ; Ratusky et Sorm, Collect. volume 23, (1958) pages 467 à 476. Les produits de formule (III) sont des produits commerciaux. Les produits de formules (VII), $(IX)_1$, $(IX)_2$, $(IX)_3$, (XI), (XIV), (XVI) et (XVII) sont nouveaux et sont aussi objet de la présente invention.

Les composés de formule (I) présentent d'intéressantes propriétés fongicides les rendant susceptibles d'être utilisés pour la protection vis-à-vis des champignons pathogènes. Il peut s'agir de la protection des plantes, de la protection des locaux ou de la protection des animaux.

Ces propriétés peuvent également être utilisées en hygiène et médecine humaine et animale.

Les composés de l'invention permettent de lutter contre de très nombreux champignons phytopathogènes, notamment contre : Erisyphe graminis, Sphaerotheca macularis, Sphaerotheca fuliginea, Podosphaera leucotricha, Uncinula necator, Helminthosporium sp., Rhynchosporium sp., Pseudocercosporella herpotrichoides et Gaeumannomyces graminis, Ustilago sp., Cercospora arachidicola et Cercosporidium personatum, Cercospora sp., Botrytis cinerea, Alternaria sp., Venturia inaequalis, Plasmopara viticola, Bremia lactucae, Peronospora sp., Pseudoperonospora humuli, Pseudoperonospora cubensis, Phytophthora infestans, Phytophthora sp., Puccinia recondita, Thanatephorus cucumeris, Rhizoctonia sp. ou encore des champignons ou levures intéressant la santé humaine comme Candida albicans ou Trychophyton sp.

L'invention a donc pour objet l'utilisation à titre de fongicides des produits de formule (I) telle que définie précédemment.

L'invention a de plus pour objet les compositions fongicides comprenant à titre de principe actif, au moins un des produits de formule (I) telle que définie précédemment.

Les compositions selon l'invention sont préparées selon les procédés usuels des industries agrochimique et agrovétérinaire. Ces compositions peuvent se présenter sous forme de poudres, granulés, suspensions, émulsions, solutions ou autres préparations employées classiquement pour l'utilisation de ce genre de composés.

Outre le principe actif, ces compositions contiennent en général, un véhicule et/ou un agent tensio-actif ionique ou non ionique, assurant une dispersion uniforme des substances constitutives du mélange. Le véhicule utilisé peut être un liquide, tel que l'eau, l'alcool, les hydrocarbures ou autres solvants organiques, une huile minérale, animale ou végétale, une poudre telle que le talc, les argiles, les silicates, le kieselghur.

Les exemples suivants et les études biologiques suivants illustrent l'invention sans toutefois la limiter.

**<u>EXEMPLE 1</u> : 2-isopropyl alpha-[(Z)-méthoxy méthylène] 4-(phénoxyméthyl) 5-thiazolacétate de méthyle.**

<u>Stade A</u> : (E)-4-oxo 5-phénoxy pentanoate de méthyle.

On ajoute à 0-5°C une solution contenant 55 g de 5-bromo 4-oxo pentanoate de méthyle (dont la préparation est décrite plus loin) dans 110 $cm^3$ d'acétone, à une solution de 32 g de phénol, 42 g de carbonate de potassium dans 400 $cm^3$ d'acétone, et maintient sous agitation à cette température pendant deux heures. On filtre et concentre à un demi volume puis verse de l'eau et extrait à l'éther diéthylique ; la phase organique est séchée et filtrée et le filtrat amené à sec. Le résidu obtenu est repris dans du chlorure de méthylène puis séché et amené à sec ; après chromatographie sur silice en éluant par le chlorure de méthylène, on obtient 30 g du produit recherché. Chromatographie sur couche mince ; Rf = 0,37 (éluant : chlorure de méthylène).
Analyse Infrarouge ($CHCl_3$)
ester non conjugué : 1722 et 1736 $cm^{-1}$
cycle aromatique : 1590, 1599, et 1498 $cm^{-1}$

Analyse RMN ($CDCl_3$)

| | |
|---|---|
| O-$CH_3$ | : 3,67 (s) ppm |
| CO-$CH_2$-$CH_2$-CO | : 2,65 (t) et 2,90 (t) ppm |
| O-$CH_2$-CO | : 4,60 (t) ppm |
| protons aromatiques | : 6,89 (d), 6,99 (t) et 7,30 (t) ppm |

Stade B : 5-phénoxy 4-[(triméthylsilyl) oxy] 3-pentènoate de méthyle.

On mélange sous azote 40 g du produit préparé au stade précédent, 600 $cm^3$ d'éther diéthylique et 27,5 $cm^3$ de chloro triméthylsilane. On refroidit à 0°C puis ajoute 32 $cm^3$ de 1,5-diaza 5-bicyclo[5.4.0]undécène (DBU) ; on agite en laissant remonter la température à 20-25°C pendant une heure et demie.

Après filtration, l'éther est évaporé et on récupère 48 g du produit recherché. ($Eb_{0,05}$) = 138-140°C. Chromatographie sur couche mince ; Rf = 0,5 [éluant : hexane-acétate d'éthyle (7-3)].

Stade C : 3-bromo 4-oxo 5-phénoxy pentanoate de méthyle.

On refroidit à 0°C sous azote, un mélange comprenant 6,9 g du produit préparé au stade précédent et 70 $cm^3$ de tétrahydrofuranne et on ajoute en 10 minutes 4,6 g de N-bromo succinimide, laisse sous agitation pendant une heure à cette température, puis amène à sec. On reprend à l'éther isopropylique filtre et amène à sec le filtrat sous pression réduite. On recueille ainsi le dérivé bromé qui est utilisé tel quel au stade suivant.

Stade D : 2-isopropyl 4-(phénoxméthyl) 5-thiazolacétate de méthyle.

On mélange l'intégralité du produit obtenu au stade précédent avec 50 $cm^3$ de méthanol et 3,1 g de 2-méthyl propanethioamide dont la préparation est décrite dans la demande de brevet européen publiée sous le numéro 0402246, porte au reflux pendant deux heures, verse le mélange sur de l'eau, neutralise par addition d'une solution aqueuse de bicarbonate de sodium, extrait à l'éther isopropylique, sèche et amène à sec. Le résidu est chromatographié sur silice en éluant par le mélange hexane-acétate d'éthyle (7-3). On recueille ainsi après évaporation des solvants, 3 g du produit recherché. Chromatographie sur couche mince ; Rf = 0,15 [éluant : hexane-acétate d'éthyle (7-3)]. Analyse RMN (250 MHz)

Analyse Infrarouge

| | |
|---|---|
| $CH_3$ de l'ester | : 1438 $cm^{-1}$ |
| C=O | : 1740 $cm^{-1}$ |
| cycle aromatique, et hétérocycle | : 1497, 1588, et 1599 $cm^{-1}$. |

Analyse RMN (250 MHz)

| | |
|---|---|
| -CH($CH_3$)$_2$ | : 1,40 (d) ppm ; |
| -CH($CH_3$)$_2$ | : 3,30 (m) ppm ; |
| -O-($CH_3$) | : 3,69 (s) ppm ; |
| -$CH_2$-CO | : 3,90 (s) ppm ; |
| -O-$CH_2$- | : 5,14 (s) ppm ; |
| protons aromatiques | : 6,96 (m) et 7,28 (m) ppm. |

Stade E : alpha-[(diméthylamino) méthylène] 2-isopropyl 4-(phénoxyméthyl) 5-thiazolacétate de méthyle.

On porte à 50°C un mélange contenant 3 g du produit préparé au stade précédent et 20 $cm^3$ d'acétal diéthylique du diméthylformamide ; après seize heures à cette température. Le milieu réactionnel est ensuite amené à sec et le résidu chromatographié sur silice en éluant par le mélange hexane-acétate d'éthyle (4-6). On recueille 2,5 g du produit recherché, utilisé tel quel pour le stade suivant. Chromatographie sur couche mince ; Rf = 0,37 [éluant : hexane-acétate d'éthyle (4-6)].

Stade F : alpha-[(Z)-(hydroxy méthylène)] 2-isopropyl 4-(phénoxyméthyl) 5-thiazolacétate de méthyle.

On mélange 2,3 g de l'ènamine préparée au stade précédent, 40 $cm^3$ de tétrahydrofuranne et 5 $cm^3$ d'acide chlorhydrique 2N et 13 $cm^3$ d'eau, laisse pendant trois heures à 20-25°C, évapore le tétrahydrofuranne, extrait

au chlorure de méthylène, sèche et amène à sec sous vide, reprend à l'éther et essore. On obtient ainsi 1,2 g du produit recherché.
F = 127,7°C.
Chromatographie sur couche mince ; Rf = 0,20 [éluant : hexane-acétate d'éthyle (7-3)].
Analyse Infrarouge.
C=O : 1699 $cm^{-1}$
C=C : 1640 $cm^{-1}$.
Analyse RMN (250 MHz)
$-CH(CH_3)_2$ : 1,42 (d) ppm ;
$-CH(CH_3)_2$ : 3,33 ppm ;
$-O-(CH_3)$ : 3,76 (s) ppm ;
$-O-CH_2-$ : 5,01 (s) ppm ;
protons aromatiques : 6,90 à 7,00 et 7,20 à 7,30 ppm.
=C-H : 7,42 (d,J=13Hz ; s après échange) ppm
-O-H : 12,17 (d,J=13 Hz) ppm

Stade G : 2-isopropyl alpha-[(Z)-méthoxy méthylène] 4-(phénoxyméthyl) 5-thiazolacétate de méthyle.

A une solution de 1,2 g du produit préparé au stade précédent, dans 50 $cm^3$ d'acétone et 3 $cm^3$ de tétrahydrofuranne on ajoute 4 $cm^3$ g d'iodure de méthyle et 4 g de carbonate de potassium, agite trois heures à 20-25°C, essore, amène à sec. Le résidu est chromatographié sur silice en éluant par le mélange hexane-acétate d'éthyle (7-3) ; on recueille 1,04 g du produit recherché.
Chromatographie sur couche mince ; Rf = 0,30 [éluant : hexane-acétate d'éthyle (7-3)].
Analyse RMN ($CDCl_3$, 250 MHz)
$-CH(CH_3)_2$ : 1,41 (d) ppm ;
$-CH(CH_3)_2$ : 3,31 (m) ppm ;
$CO-O-(CH_3)$ et $C=CH-O-(CH_3)$ : 3,69 (s) et 3,79 (s) ppm ;
$-O-CH_2-$ : 4,97 (s) ppm ;
protons aromatiques : 6,88 à 6,95 et 7,20 à 7,30 ppm.
Microanalyse pour $C_{18}H_{21}NO_4S$ = 347,432

| | | | | |
|---|---|---|---|---|
| calculés : | C% 62,23 | H% 6,09 | N% 4,03 | S% 9,23 |
| trouvés : | 62,4 | 6,2 | 4,1 | 9,1 |

**EXEMPLE 2 : alpha-[(Z)-méthoxy méthylène] 2-méthyl 4-(phénoxy-méthyl) 5-thiazolacétate de méthyle.**

Stade A : 2-Méthyl 4-(phénoxyméthyl) 5-thiazolacétate de méthyle.

On mélange 4,6 g du produit obtenu au stade B de l'exemple 1 avec 45 $cm^3$ de tétrahydrofuranne, refroidit à 0°C puis introduit 3,1 g de N-bromo succinimide, agite pendant 45 minutes à cette température et évapore le tétrahydrofuranne sous pression réduite ; on reprend à l'éther isopropylique filtre le produit cristallisé et amène le filtrat à sec. Le résidu huileux est repris par 30 $cm^3$ de méthanol et on ajoute 1,5 g de thioacétamide. Après deux heures au reflux, on concentre sous vide, dissout le résidu dans une solution aqueuse de bicarbonate de sodium, extrait à l'éther, lave la phase organique par une solution saturée de chlorure de sodium, sèche et amène à sec. Le résidu est chromatographié sur silice en éluant par le mélange hexane-acétate d'éthyle (75-25). On recueille ainsi après évaporation des solvants, 3,3 g du produit recherché.
Chromatographie sur couche mince ; Rf = 0,12 [éluant : hexane-acétate d'éthyle (75-25)].
Analyse RMN ($CDCl_3$, 250 MHz)
$-CH_3$ : 2,68 (s) ppm ;
$-O-(CH_3)$ : 3,68 (s) ppm ;
$-CH_2-CO$ : 3,88 (s) ppm ;
$-O-CH_2-$ : 5,12 (s) ppm ;
protons aromatiques : 6,98 (m) et 7,28 (m) ppm.

Stade B : alpha-[(diméthylamino) méthylène] 2-méthyl 4-(phénoxyméthyl) 5-thiazolacétate de méthyle.

On porte à 50-70°C un mélange contenant 1,6 g du produit préparé au stade précédent et 20 $cm^3$ d'acétal diéthylique du diméthylformamide ; après trois heures à cette température. Le milieu réactionnel est amené à sec et le résidu chromatographié sur silice en éluant par le mélange cyclohexane-acétate d'éthyle (6-4). On recueille 1,3 g du produit recherché. Chromatographie sur couche mince ; Rf = 0,15 [éluant : cyclohexane-acétate d'éthyle (6-4)].

Analyse RMN ($CDCl_3$, 250 MHz)

| | |
|---|---|
| $-C\underline{H}_3$ | : 2,70 (s) ppm ; |
| $-N(C\underline{H}_3)_2$ | : 2,79 (s) ppm ; |
| $-O-C\underline{H}_3$ | : 3,63 (s) ppm ; |
| $-O-C\underline{H}_2-$ | : 4,91 ppm ; |
| protons aromatiques | : 6,90 (m) et 7,26 (m) ppm. |
| =C-$\underline{H}$ | : 7,60 ppm |

Microanalyse pour $C_{17}H_{20}N_2O_3S$ = 322,421

| | | | | |
|---|---|---|---|---|
| calculés : | C% 61,42 | H% 6,06 | N% 8,43 | S% 9,64 |
| trouvés : | 61,4 | 6,1 | 8,5 | 9,7 |

Stade C : alpha-[(Z)-(hydroxy méthylène)] 2-méthyl 4-(phénoxyméthyl) 5-thiazolacétate de méthyle.

On mélange 2,5 g de l'ènamine préparée au stade précédent, 40 $cm^3$ de tétrahydrofuranne, 5 $cm^3$ d'acide chlorhydrique 2N et 13 $cm^3$ d'eau, laisse pendant quatre heures à 20-25°C et neutralise par addition de bicarbonate de sodium ; on extrait au chlorure de méthylène, lave la phase organique, sèche et amène à sec sous vide, et recristallise dans l'éther, essore et sèche.

On obtient ainsi 2,15 g du produit recherché.

Chromatographie sur couche mince ; Rf = 0 à 0,10 [éluant : cyclohexane-acétate d'éthyle (6-4)].

Analyse RMN ($CDCl_3$, 250 MHz)

| | |
|---|---|
| $-C\underline{H}_3$ | : 2,69 (s) ppm ; |
| $-O-(C\underline{H}_3)$ | : 3,76 (s) ppm ; |
| $-O-C\underline{H}_2-$ | : 4,97 (s) ppm ; |
| protons aromatiques | : 6,95 (m) et 7,29 (m) ppm. |
| =C-$\underline{H}$ | : 7,40 (d,s après échange) |
| -O-$\underline{H}$ | : 12,14 (d,mobile) ppm |

Stade D : alpha-[(Z)-méthoxy méthylène] 2-méthyl 4-(phénoxyméthyl) 5-thiazolacétate de méthyle.

A une solution de 2 g du produit préparé au stade précédent, dans 70 $cm^3$ d'acétone on ajoute 7 $cm^3$ d'iodure de méthyle et 3,5 g de carbonate de potassium, le tout est agité 30 minutes à 20-25°C, essoré, amené à sec et le résidu est chromatographié sur silice en éluant par le mélange hexaneacétate d'éthyle (1-1) ; on recueille 1,41 g du produit recherché.

Chromatographie sur couche mince ; Rf = 0,27 [éluant : hexane-acétate d'éthyle (1-1)].

Analyse RMN ($CDCl_3$, 250 MHz)

| | |
|---|---|
| $-CH_3$ | : 2,70 (s) ppm ; |
| $=C\underline{H}-O$ | : 7,88 (s) ppm ; |
| $CO-O-(C\underline{H}_3)$ et $C=CH-O-(C\underline{H}_3)$ | : 3,69 (s) et 3,80 (s) ppm ; |
| $-O-C\underline{H}_2-$ | : 4,95 (s) ppm ; |
| protons aromatiques | : 6,92 (m) à 7,24 (m) ppm. |

Microanalyse pour $C_{18}H_{21}NO_4S$ = 319,382

| | | | | |
|---|---|---|---|---|
| calculés : | C% 60,17 | H% 5,36 | N% 4,39 | S% 10,04 |
| trouvés : | 59,4 | 5,3 | 4,3 | 10,0 |

**<u>EXEMPLE 3</u> : alpha-[(Z)-méthoxy méthylène] 4-(phénoxyméthyl) 5-thiazolacétate de méthyle.**

<u>Stade A</u> : 4-(phénoxyméthyl) 5-thiazolacétate de méthyle.

On mélange 13,8 g du produit obtenu au stade B de l'exemple 1 avec 135 $cm^3$ de tétrahydrofuranne, refroidit à 0°C puis introduit 9,3 g de N-bromo succinimide, agite pendant une heure à cette température et élimine le tétrahydrofuranne ; on reprend à l'éther isopropylique filtre le produit cristallisé et amène le filtrat à sec. Le résidu huileux est dissout dans 100 $cm^3$ de méthanol et on ajoute 3,0 g de méthanethioamide. Après deux heures au reflux, on verse le mélange sur de l'eau, neutralise par une solution aqueuse saturée de bicarbonate de sodium, extrait à l'éther isopropylique, lave, sèche et amène à sec. Le résidu est chromatographié sur silice en éluant par le mélange hexane-acétate d'éthyle (7-3). On recueille ainsi après évaporation des solvants, 5 g du produit recherché. Chromatographie sur couche mince ; Rf = 0,2 [éluant : hexane-acétate d'éthyle (7-3)].
Analyse RMN ($CDCl_3$, 250 MHz)

| | |
|---|---|
| -O-($CH_3$) | : 3,69 (s) ppm ; |
| -$C\underline{H}_2$-CO | : 3,97 (s) ppm ; |
| -O-$C\underline{H}_2$- | : 5,23 (s) ppm ; |
| protons aromatiques | : 6,96 (m) et 7,28 (m) ppm. |
| N=C-H | : 8,70 (s) ppm |

<u>Stade B</u> : alpha-[(diméthylamino) méthylène] 4-(phénoxyméthyl) 5-thiazolacétate de méthyle.

On chauffe pendant deux heures à 50°C, un mélange contenant 5 g du produit préparé au stade précédent et 30 $cm^3$ d'acétal diéthylique du diméthylformamide, amène à sec et le résidu est chromatographié sur silice en éluant par le mélange hexane-acétate d'éthyle (4-6). On recueille 3 g du produit recherché, utilisé tel quel pour le stade suivant.
Chromatographie sur couche mince ; Rf = 0,3 [éluant : hexane-acétate d'éthyle (4-6)].

<u>Stade C</u> : alpha-[(Z)-(hydroxy méthylène)] 4-(phénoxyméthyl) 5-thiazolacétate de méthyle.

On mélange 3 g de l'ènamine préparée au stade précédent, 50 $cm^3$ de tétrahydrofuranne et 5 $cm^3$ d'acide chlorhydrique 2N et 15 $cm^3$ d'eau, laisse pendant deux heures et demi à 20-25°C, évapore le tétrahydrofuranne, extrait au chlorure de méthylène, lave la phase organique, sèche et amène à sec sous vide, puis recristallise dans l'éther isopropylique, essore et sèche.

On obtient ainsi 2,5 g du produit recherché. F = 133°C.
Chromatographie sur couche mince ; Rf = 0,25 [éluant : hexane-acétate d'éthyle (7-3)].
Analyse RMN (DMSO, 250 MHz)

| | |
|---|---|
| -O-($C\underline{H}_3$) | : 3,61 (s) ppm ; |
| -O-$C\underline{H}_2$- | : 4,96 (s) ppm ; |
| protons aromatiques | : 6,90 (m) et 7,25 (m) ppm. |
| =C-<u>H</u> | : 7,98 (s) ppm |
| N=C-<u>H</u> | : 9,06 (s) ppm |
| -O-<u>H</u> | : 11,77 (mobile) ppm |

<u>Stade D</u> : alpha-[(Z)-méthoxy méthylène] 4-(phénoxméthyl) 5-thiazolacétate de méthyle.

A une solution de 2,5 g du produit préparé au stade précédent dans 100 $cm^3$ d'acétone et 20 $cm^3$ de tétrahydrofuranne on ajoute 8 $cm^3$ d'iodure de méthyle et 4 g de carbonate de potassium, le tout est agité quatre heures à 20-25°C, filtré, le filtrat amené à sec et le résidu chromatographié sur silice en éluant par le mélange hexane-acétate d'éthyle (4-6) ; on recueille 1,7 g du produit recherché.
Chromatographie sur couche mince ; Rf = 0,35 [éluant : hexane-acétate d'éthyle (4-6)].
Analyse RMN (250 MHz)

| | |
|---|---|
| CO-O-($C\underline{H}_3$) et C=CH-O-($C\underline{H}_3$) | : 3,69 (s) et 3,81 (s) ppm ; |
| -O-$C\underline{H}_2$- | : 5,07 (s) ppm ; |
| protons aromatiques | : 6,88 à 6,96 et 7,19 à 7,27 ppm. |
| C=C-<u>H</u> | : 7,62 (s) ppm |
| N=C-<u>H</u> | : 8,82 (s) ppm |

Microanalyse pour $C_{15}H_{15}NO_4S$ = 305,356

| | | | | | |
|---|---|---|---|---|---|
| calculés | : | C% 59,00 | H% 4,95 | N% 4,59 | S% 10,5 |
| trouvés | : | 58,5 | 4,9 | 4,5 | 10,5 |

**EXEMPLE 4 : 2-chloro alpha-[(Z)-méthoxy méthylène] 4-(phénoxyméthyl) 5-thiazolacétate de méthyle.**

Stade A : 2,3-dihydro 2-oxo 4-(phénoxyméthyl) 5-thiazolacétate de méthyle.

On mélange 18 g du produit préparé au stade B de l'exemple 1 avec 180 $cm^3$ de tétrahydrofuranne, refroidit à 0/+5°C puis ajoute 12 g de N-bromo succinimide, agite pendant une heure, amène à sec, reprend à l'éther isopropylique, filtre et amène le filtrat à sec sous vide.

L'huile obtenue est reprise avec 150 $cm^3$ de méthanol puis on ajoute 7 g de thiocarbamate d'éthyle, chauffe au reflux pendant 3 heures, puis verse dans l'eau, extrait au chlorure de méthylène, sèche et amène à sec. Après cristallisation dans l'éther isopropylique, on obtient 8,3 g du produit recherché F = 103°C.
Chromatographie sur couche mince ; Rf = 0,42 [éluant : hexane-acétate d'éthyle (1-1)].
Analyse RMN (250 MHz) ppm

| | |
|---|---|
| $-CO_2-C\underline{H}_3$ | : 3,72 (s) |
| $-CO-C\underline{H}_2$ | : 3,53 (s) |
| $O-C\underline{H}_2-$ | : 4,79 (s) |
| NH | : 9,80 (mobile) |
| aromatiques | : 6,90 à 7,05 (s) et 7,25 à 7,35 (m). |

Stade B : 2-chloro 4-(phénoxyméthyl) 5-thiazolacétate de méthyle.

12 g du produit préparé au stade précédent sont mélangés à 24 $cm^3$ de chlorure de phosphoryle, puis on introduit goutte à goutte 5 $cm^3$ de 2,6-lutidine. Après chauffage au reflux pendant trois heures, on laisse revenir le mélange à 20-25°C. On chasse l'excès de chlorure de phosphoryle sous pression réduite et verse le milieu réactionnel sur 80 $cm^3$ d'eau glacée et extrait au chlorure de méthylène ; la phase organique est lavée, séchée et amenée à sec et le résidu est chromatographié sur silice en éluant par le mélange hexane-acétate d'éthyle (7-3) ; on recueille après évaporation des solvants, 9 g du produit recherché.
Chromatographie sur couche mince ; Rf = 0,32 [éluant : hexane-acétate d'éthyle (7-3)].
Spectre IR :

| | |
|---|---|
| C = O | : 1740 $cm^{-1}$ |
| Aromatique + | : 1598 - 1590 |
| Système conjugué | : 1496 - 1488 $cm^{-1}$ |

Stade C : 2-chloro alpha-[(diméthylamino) méthylène] 4-(phénoxyméthyl) 5-thiazolacétate de méthyle.

On chauffe à 50°C pendant trois heures un mélange contenant 4 g du produit préparé au stade précédent et 50 $cm^3$ d'acétal diéthylique du diméthylformamide, amène à sec et le résidu est chromatographié sur silice en éluant par le mélange hexane-acétate d'éthyle (7-3) ; on recueille 1,5 g du produit recherché. F = 113,8°C.
Chromatographie sur couche mince ; Rf = 0,25 [éluant : hexane-acétate d'éthyle (7-3)].

Stade D : 2-chloro alpha-[hydroxy méthylène] 4-(phénoxyméthyl) 5-thiazolacétate de méthyle.

2,2 g du produit préparé au stade précédent sont mélangés à 50 $cm^3$ de tétrahydrofuranne puis 4 $cm^3$ d'acide chlorhydrique 2N et 10 $cm^3$ d'eau, et le tout est laissé à 20-25°C pendant deux heures. Le solvant est évaporé sous pression réduite, et le milieu extrait au chlorure de méthylène ; la phase organique est séchée et amenée à sec ; on obtient 2,1 g du produit recherché.
Chromatographie sur couche mince ; Rf = 0,1 à 0,3 [éluant : hexane-acétate d'éthyle (7-3)].
Spectre IR :

| | |
|---|---|
| C = O | : 1664 $cm^{-1}$ ester chelaté |
| C = C + | : 1599 - 1590 |
| Aromatique + | : 1544 - 1496 $cm^{-1}$ |
| Hétérocycle | |

Stade E : 2-chloro alpha-[(Z)-méthoxy méthylène] 4-(phénoxyméthyl) 5-thiazolacétate de méthyle.

A une solution de 2 g du produit obtenu obtenu au stade précédent dans 50 $cm^3$ d'acétone on ajoute 8 $cm^3$ d'iodure de méthyle et 4 g de carbonate de potassium et agite quatre heures et demi à 20-25°C. Après neutralisation par une solution aqueuse d'acide chlorhydrique N, le milieu est extrait au chlorure de méthylène, la phase organique lavée, sèchée et amenée à sec ; le résidu est chromatographié sur silice en éluant par le mélange hexane-acétate d'éthyle (7-3). On recueille après évaporation des solvants, 1,5 g du produit recherché.
Chromatographie sur couche mince ; Rf = 0,25 [éluant : hexane-acétate d'éthyle (6-4)].
Analyse RMN

| | |
|---|---|
| CO-O-($CH_3$) et C=CH-O-($CH_3$) | : 3,70 (s) et 3,82 (s) ppm ; |
| -O-$CH_2$- | : 4,95 (s) ppm ; |
| protons aromatiques | : 6,8 à 7,0 (m) et 7,2 à 7,4 (m)ppm; |
| C=C-H | : 7,58 (s) ppm. |

Microanalyse pour $C_{15}H_{14}ClNO_4S$ = 334,796

| | | | | | |
|---|---|---|---|---|---|
| calculés : | C% 53,02 | H% 4,15 | Cl% 10,43 | N% 4,12 | S% 9,45 |
| trouvés : | 52,9 | 4,1 | 10,6 | 4,1 | 9,5 |

**EXEMPLE 5 : alpha-[(Z)-méthoxy méthylène] 2-méthylthio 4-(phénoxyméthyl) 5-thiazolacétate de méthyle.**

Stade A : 4-(phénoxyméthyl) 2-thioxo 5-thiazolacétate de méthyle.

A une solution de 9 g du produit préparé au stade B de l'exemple 1 dans 90 $cm^3$ de tétrahydrofuranne, refroidi à 0°C on ajoute 6 g de N-bromo succinimide et le tout est agité une heure à cette température ; on amène à sec, et reprend le résidu à l'éther isopropylique, essore la succinimide puis amène le filtrat à sec ; l'huile obtenue est dissoute dans 70 $cm^3$ de méthanol puis traitée par 3,5 g de dithiocarbamate d'ammonium pendant une heure au reflux ; on verse ensuite dans l'eau, essore le produit, le reprend dans le chlorure de méthylène, le sèche et amène à sec sous vide. Après cristallisation dans l'éther isopropylique, on recueille 3,6 g du produit recherché. F = 137,8°C
Chromatographie sur couche mince ; Rf = 0,40 [éluant : chlorure de méthylène-acétate d'éthyle (9-1)].
Spectre IR :

| | |
|---|---|
| C = NH | : 3385 $cm^{-1}$ |
| $CO_2$-$CH_3$ | : 1742 - 1438 $cm^{-1}$ |
| C=C | : 1625 $cm^{-1}$ |

Stade B : alpha-[(diméthylamino) méthylène] 4-méthylthio 4-(phénoxyméthyl) 5-thiazolacétate de méthyle.

On chauffe à 50°C pendant deux heures un mélange contenant 3,4 g du produit préparé au stade précédent et 25 $cm^3$ d'acétal diéthylique du diméthylformamide, amène à sec et le résidu est chromatographié sur silice en éluant par le mélange hexane-acétate d'éthyle (6-4) ; on recueille 2,1 g du produit recherché. F = 122,1°C
Chromatographie sur couche mince ; Rf = 0,22 [éluant : hexane-acétate d'éthyle (6-4)].

Stade C : alpha-[hydroxy méthylène] 4-méthylthio 4-(phénoxyméthyl) 5-thiazoleacétate de méthyle.

2,1 g du produit préparé au stade précédent sont mélangés à 45 $cm^3$ de tétrahydrofuranne puis 3,4 $cm^3$ d'acide chlorhydrique 2N et le tout est laissé à 20-25°C pendant seize heures. Le solvant est évaporé sous vide, et le milieu extrait au chlorure de méthylène ; la phase organique est séchée et amenée à sec ; on obtient 1,89 g du produit recherché. Chromatographie sur couche mince ; Rf = 0,25 [éluant : hexane-acétate d'éthyle (4-6)].
Spectre IR :

| | |
|---|---|
| C = O | : 1701 - 1666 $cm^{-1}$ |
| C = C + | : 1635 - 1618 |
| Aromatique + | : 1598 - 1590 |
| Hétérocycle | : 1548 - 1497 $cm^{-1}$ |

Stade D : alpha-[(Z)-méthoxy méthylène] 4-méthylthio 4-(phénoxyméthyl) 5-thiazolacétate de méthyle.

1,8 g du produit obtenu obtenu au stade précédent sont dissouts dans 70 $cm^3$ d'acétone, et traités avec 7 $cm^3$ d'iodure de méthyle et 4 g de carbonate de potassium sous agitation pendant une heure et quinze minutes à 20-25°C ; le mélange est versé dans l'eau, neutralisé par ajout d'acide chlorhydrique 2N, extrait au chlorure de méthylène, la phase organique lavée, sèchée et amenée à sec ; le résidu est chromatographié sur silice en éluant par le mélange hexane-acétate d'éthyle (7-3). On recueille après évaporation des solvants, 1,56 g du produit recherché.
Chromatographie sur couche mince ; Rf = 0,25 [éluant : hexane-acétate d'éthyle (7-3)].
Analyse RMN ($CDCl_3$ 250MHz)

| | |
|---|---|
| S-$C\underline{H}_3$ | : 2,66 (s) |
| CO-O-($C\underline{H}_3$) et C=CH-O-($C\underline{H}_3$) | : 3,68 (s) et 3,79 (s) ppm ; |
| -O-$C\underline{H}_2$- | : 4,96 (s) ppm ; |
| protons aromatiques | : 6,9 à 6,93 et 7,19 à 7,27 ppm. |
| C=C-$\underline{H}$ | : 7,56 (s) ppm |

Microanalyse pour $C_{16}H_{17}NO_4S_2$ : 351,438

| | | | | |
|---|---|---|---|---|
| calculés : | C% 54,68 | H% 4,88 | N% 3,99 | S% 18,24 |
| trouvés : | 54,6 | 4,8 | 4,1 | 18,1 |

**Préparation du 5-bromo 4-oxo pentanoate de méthyle**

Stade A : 4-oxo pentanoate de méthyle.

On agite pendant seize heures à 20-25°C, une solution contenant 300 g d'acide 4-oxo pentanoïque (acide lévulinique), 300 $cm^3$ de méthanol et 2 $cm^3$ d'acide sulfurique à 97 %, amène à sec et rectifie le résidu sous pression réduite. on recueille 290 g de l'ester méthylique attendu. $Eb_{16}$ = 87-88°C. Chromatographie sur couche mince ; Rf = 0,13 [éluant : hexane-acétate d'éthyle (8-2)].

Stade B : 5-bromo 4-oxo pentanoate de méthyle

100 g de l'ester préparé au stade précédent sont mélangés à 1600 $cm^3$ de méthanol et 2$cm^3$ d'acide acétique concentré et le tout est chauffé à 40°C ; on introduit alors 42 $cm^3$ de brome puis on laisse revenir la température à 25°C sous agitation. Le mélange est amené à sec, le résidu obtenu dissout dans 100 $cm^3$ d'eau et neutralisé avec une solution aqueuse saturée de bicarbonate de soude. Après extraction à l'éther diéthylique, la phase organique est séchée sur sulfate de magnésium, filtrée et amenée à sec. Le résidu obtenu est chromatographié sur silice et le produit recherché est recueilli avec un rendement de 34 %.
Chromatographie sur couche mince ; Rf = 0,18 [éluant : hexane-cétate d'éthyle (8-2)].

**ETUDE BIOLOGIQUE**

**ETUDE DE L'ACTIVITE FONGICIDE**

Essais sur Puccinia recondita tritici

Les grains de blé (variété Festival) sont mis à germer dans le mélange terre-terreau-sable (1/3,1/3,1/3). Les plants sont cultivés en serre. Les produits sont dissous dans la "matrice A" juste avant l'essai, à une concentration de 500 ppm. Le traitement s'effectue par pulvérisation de la solution de produit sur les plants de blé âgés de neuf jours, jusqu'à rétention maximale. La contamination par les urédospores de Puccinia recondita tritici est faite le lendemain du traitement. Les plants sont maintenus en salle climatisée (température de jour : 22°C, température de nuit : 18°C). Sept jours après la contamination, on mesure la densité des spores sur les deux premières feuilles de chaque plant. L'efficacité du produit est calculée par rapport à un témoin non traité.
Résultats :

| EX. | Essai |
|---|---|
| 3 | 100 % |
| 4 | 80 % |

<u>Exemple de compositions</u>.

| | | |
|---|---|---|
| Matrice A : | SOLVESSO 150 | 70,0 g |
| | NAPSOL PM1 | 850,0 g |
| | SURFAROX HRH 40C | 52,0 g |
| | ECD 1604 | 28,0 g |
| | | ---------- |
| | | 1000,0 g |

**Revendications**

**1)** Les produits de formule (I) :

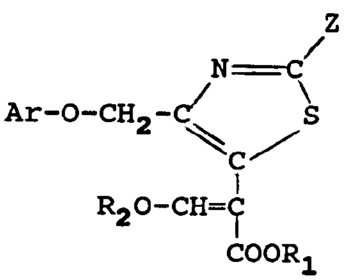

(I)

dans laquelle Ar représente un radical phényle non substitué ou substitué par un ou plusieurs radicaux identiques ou différents choisis parmi les atomes d'halogène, le radical méthylènedioxy, le radical phénoxy, le radical phényle, le radical trifluorométhyle, les radicaux alkyle linéaires ou ramifiés renfermant de un à six atomes de carbone, les radicaux alkyloxy linéaires ou ramifiés renfermant de un à six atomes de carbone ou les radicaux alkylthio linéaires ou ramifiés renfermant de un à six atomes de carbone, Z représente un atome d'hydrogène, un atome de chlore, un radical trifluorométhyle, un radical alkyle linéaire ou ramifié renfermant de un à six atomes de carbone, un radical alkyloxy linéaire ou ramifié renfermant de un à six atomes de carbone, un radical alkylthio linéaire ou ramifié renfermant de un à six atomes de carbone, $R_1$ et $R_2$ identiques ou différents représentent indépendamment l'un de l'autre, un radical alkyle linéaire ou ramifié renfermant de un à six atomes de carbone et la double liaison exocyclique est en configuration (Z) ou en configuration (E).

**2)** Les produits de formule (I) telle que définie à la revendication 1 dans laquelle $R_1$ et $R_2$ représentent un radical méthyle.

**3)** Les produits de formule (I) telle que définie à l'une quelconque des revendications 1 ou 2 dans laquelle Z représente un atome d'hydrogène, un atome de chlore ou un des radicaux choisis parmi les radicaux méthyle, éthyle, propyle, isopropyle, méthoxy, éthoxy et méthylthio.

**4)** Les produits dont les noms suivent :

– alpha-[(Z)-méthoxy méthylène] 4-(phénoxyméthyl) 5-thiazolacétate de méthyle,

– 2-chloro alpha-[(Z)-méthoxy méthylène] 4-(phénoxyméthyl) 5-thiazolacétate de méthyle.

**5)** Procédé de préparation des produits de formule (I) telle que définie à la revendication I caractérisé en

ce qu'un ester de l'acide 5-halo 4-oxo pentanoïque de formule (II) :

$X\text{-}CH_2\text{-}CO\text{-}CH_2\text{-}CH_2\text{-}COOR_1$ (II)

dans laquelle X représente un atome de chlore ou de brome et $R_1$ a la signification indiquée à la revendication 1, est traité en présence d'une base avec un phénol de formule (III) :

Ar-OH (III)

dans laquelle Ar a la signification indiquée à la revendication 1, pour obtenir l'ester de l'acide 5-(aryloxy) 4-oxo pentanoïque de formule (IV) :

$Ar\text{-}O\text{-}CH_2\text{-}CO\text{-}CH_2\text{-}CH_2\text{-}COOR_1$ (IV)

ester de formule (IV), que l'on transforme par action d'un trialkyl chloro silane de formule (V) :

$R_3R_4R_5SiCl$ (V)

dans laquelle $R_3$, $R_4$ et $R_5$ identiques ou différents, représentent un radical alkyle linéaire ou ramifié renfermant de un à quatre atomes de carbone, en un ether d'énol silylé de formule (VI) :

$Ar\text{-}O\text{-}CH_2\text{-}C(OSiR_3R_4R_5)\text{=}CH\text{-}CH_2\text{-}COOR_1$ (VI)

produit de formule (VI) qui, par bromation permet d'obtenir le dérivé 3-bromo de l'ester de l'acide 5-(aryloxy) 4-oxo pentanoïque de formule (VII) :

$Ar\text{-}O\text{-}CH_2\text{-}CO\text{-}CHBr\text{-}CH_2\text{-}COOR_1$ (VII)

que l'on fait réagir :

soit avec le produit de formule (VIII) :

$Z_1\text{-}CS\text{-}NH_2$ (VIII)

dans laquelle $Z_1$ représente un atome d'hydrogène ou un radical alkyle linéaire ou ramifié, renfermant de un à six atomes de carbone, pour former un produit de formule $(IX)_1$ :

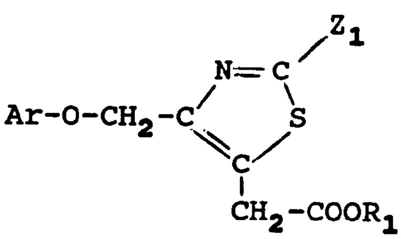

$(IX)_1$

soit avec un produit de formule (X) :

$Alc_1\text{-}O\text{-}CS\text{-}NH_2$ (X)

dans laquelle $Alc_1$ représente un radical alkyle renfermant de 1 à 4 atomes de carbone, pour former le dérivé de la 2-thiazolinone de formule (XI) :

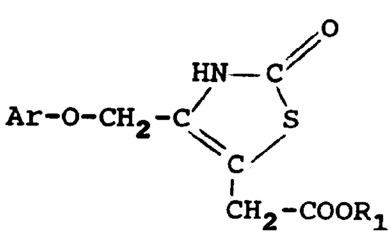

(XI)

produit de formule (XI) que l'on traite avec un réactif de chloration des fonctions carbonyle pour obtenir le produit de formule $(IX)_2$ :

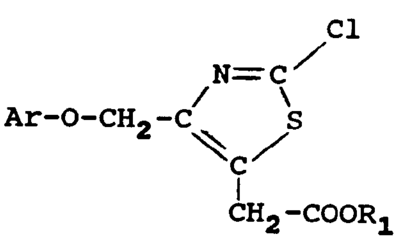

$(IX)_2$

produit de formule (XI) qui si désiré, est mis à réagir avec une base de formule (XII) :

$Z_3^-M^+$ (XII)

dans laquelle $Z_3^-$ représente un anion alcoolate et M+ un cation ammonium ou un cation de métal alcalin, pour conduire au produit de formule $(IX)_3$ :

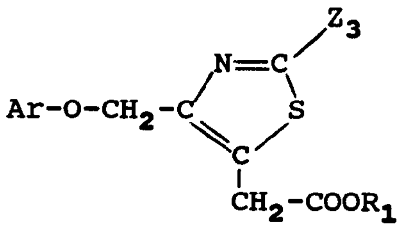

$(IX)_3$

soit avec un dithiocarbamate de formule (XIII) :

$NH_2$-CS-$S^-$ $M^+$ (XIII)

pour obtenir le dérivé de la 2-thiazolinethione de formule (XIV) :

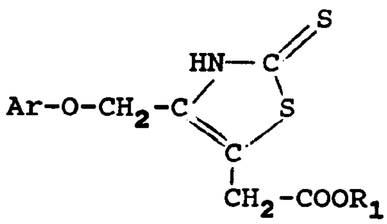

(XIV)

produits de formules $(IX)_1$, $(IX)_2$, $(IX)_3$ ou (XIV), que l'on condense en présence d'une base avec un acétal du diméthyl formamide de formule (XV) :

$Me_2N$-$CH(OR_7)_2$ (XV)

dans laquelle $R_7$ représente un radical alkyle linéaire ou ramifié renfermant de un à six atomes de carbone, pour obtenir respectivement les produits de formules $(XVI)_1$, $(XVI)_2$, $(XVI)_3$, et $(XVI)_4$ :

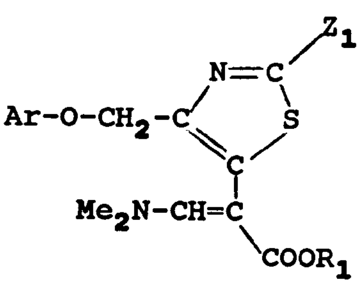

$(XVI)_1$,

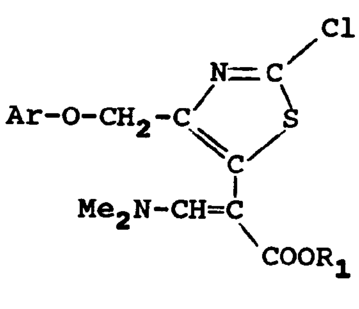

$(XVI)_2$,

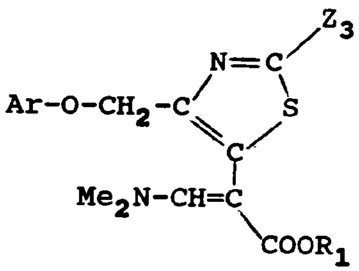

(XVI)$_3$ et

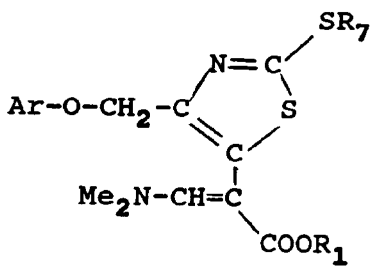

(XVI)$_4$

produits de formules (XVI)$_1$, (XVI)$_2$, (XVI)$_3$ et (XVI)$_4$ que l'on peut représenter sous la seule formule (XVI) :

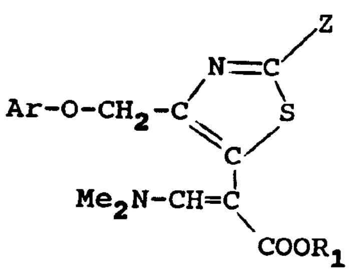

(XVI)

dans laquelle Z, Ar et $R_1$ a la signification indiquée à la revendication 1, et produit de formule (XVI) qui, par hydrolyse est transformé en produit de formule (XVII) :

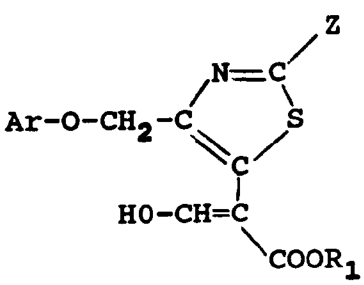

(XVII)

produit de formule (XVII) que l'on éthérifie en produit de formule (I).

**6)** Utilisation à titre de fongicides des produits de formule (I) telle que définie à l'une quelconque des revendications 1 à 4.

**7)** Compositions fongicides comprenant à titre de principe actif, au moins un des produits de formule (I) telle que définie à l'une quelconque des revendications 1 à 4.

**8)** Les produits intermédiaires du procédé décrit à la revendication 5 de formules (VII), (IX)$_1$, (IX)$_2$, (IX)$_3$, (XI), (XIV), (XVI) et (XVII).

**1)** Procédé pour préparer des produits de formule (I) :

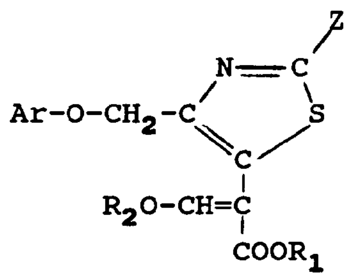

(I)

dans laquelle Ar représente un radical phényle non substitué ou substitué par un ou plusieurs radicaux identiques ou différents choisis parmi les atomes d'halogène, le radical méthylènedioxy, le radical phénoxy, le radical phényle, le radical trifluorométhyle, les radicaux alkyle linéaires ou ramifiés renfermant de un à six atomes de carbone, les radicaux alkyloxy linéaires ou ramifiés renfermant de un à six atomes de carbone ou les radicaux alkylthio linéaires ou ramifiés renfermant de un à six atomes de carbone, Z représente un atome d'hydrogène, un atome de chlore, un radical trifluorométhyle, un radical alkyle linéaire ou ramifié renfermant de un à six atomes de carbone, un radical alkyloxy linéaire ou ramifié renfermant de un à six atomes de carbone, un radical alkylthio linéaire ou ramifié renfermant de un à six atomes de carbone, $R_1$ et $R_2$ identiques ou différents représentent indépendamment l'un de l'autre, un radical alkyle linéaire ou ramifié renfermant de un à six atomes de carbone et la double liaison exocyclique est en configuration (Z) ou en configuration (E), caractérisé en ce qu'un ester de l'acide 5-halo 4-oxo pentanoïque de formule (II) :

$X\text{-}CH_2\text{-}CO\text{-}CH_2\text{-}CH_2\text{-}COOR_1$ (II)

dans laquelle X représente un atome de chlore ou de brome et $R_1$ a la signification indiquée ci-dessis, est traité en présence d'une base avec un phénol de formule (III) :

Ar-OH (III)

dans laquelle Ar a la signification indiquée ci-dessus, pour obtenir l'ester de l'acide 5-(aryloxy) 4-oxo pentanoïque de formule (IV) :

$Ar\text{-}O\text{-}CH_2\text{-}CO\text{-}CH_2\text{-}CH_2\text{-}COOR_1$ (IV)

ester de formule (IV), que l'on transforme par action d'un trialkyl chloro silane de formule (V) :

$R_3R_4R_5SiCl$ (V)

dans laquelle $R_3$, $R_4$ et $R_5$ identiques ou différents, représentent un radical alkyle linéaire ou ramifié renfermant de un à quatre atomes de carbone, en un ether d'énol silylé de formule (VI) :

$Ar\text{-}O\text{-}CH_2\text{-}C(OSiR_3R_4R_5)\text{=}CH\text{-}CH_2\text{-}COOR_1$ (VI)

produit de formule (VI) qui, par bromation permet d'obtenir le dérivé 3-bromo de l'ester de l'acide 5-(aryloxy) 4-oxo pentanoïque de formule (VII) :

$Ar\text{-}O\text{-}CH_2\text{-}CO\text{-}CHBr\text{-}CH_2\text{-}COOR_1$ (VII)

que l'on fait réagir :

soit avec le produit de formule (VIII) :

$Z_1\text{-}CS\text{-}NH_2$ (VIII)

dans laquelle $Z_1$ représente un atome d'hydrogène ou un radical alkyle linéaire ou ramifié, renfermant de un à six atomes de carbone, pour former un produit de formule $(IX)_1$ :

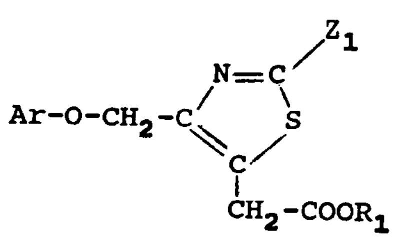

$(IX)_1$

soit avec un produit de formule (X) :

$Alc_1\text{-}O\text{-}CS\text{-}NH_2$ (X)

dans laquelle $Alc_1$ représente un radical alkyle renfermant de 1 à 4 atomes de carbone, pour former le dérivé de la 2-thiazolinone de formule (XI) :

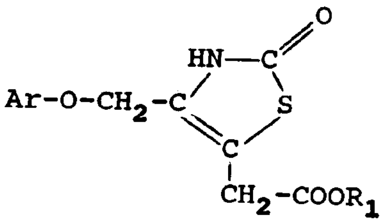

(XI)

produit de formule (XI) que l'on traite avec un réactif de chloration des fonctions carbonyle pour obtenir le produit de formule $(IX)_2$ :

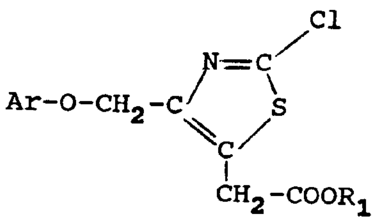

$(IX)_2$

produit de formule (XI) qui si désiré, est mis à réagir avec une base de formule (XII) :

$Z_3^- M^+$ (XII)

dans laquelle $Z_3^-$ représente un anion alcoolate et $M^+$ un cation ammonium ou un cation de métal alcalin, pour conduire au produit de formule $(IX)_3$ :

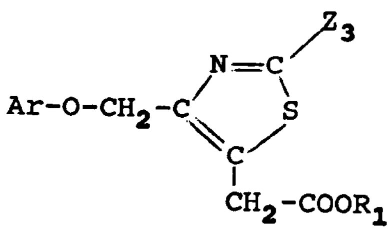

$(IX)_3$

soit avec un dithiocarbamate de formule (XIII) :

$NH_2\text{-}CS\text{-}S^- M^+$ (XIII)

pour obtenir le dérivé de la 2-thiazolinethione de formule (XIV) :

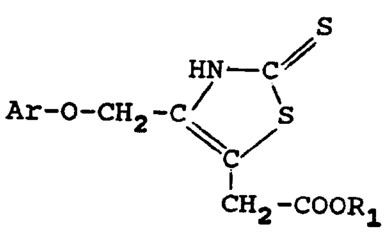

(XIV)

produits de formules $(IX)_1$, $(IX)_2$, $(IX)_3$ ou (XIV), que l'on condense en présence d'une base avec un acétal du diméthyl formamide de formule (XV) :

$Me_2N\text{-}CH(OR_7)_2$ (XV)

dans laquelle $R_7$ représente un radical alkyle linéaire ou ramifié renfermant de un à six atomes de carbone, pour obtenir respectivement les produits de formules $(XVI)_1$, $(XVI)_2$, $(XVI)_3$, et $(XVI)_4$ :

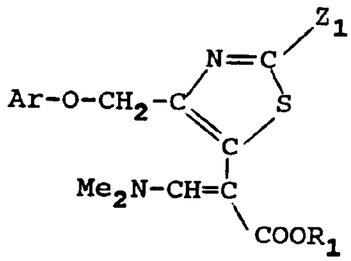

(XVI)$_1$,

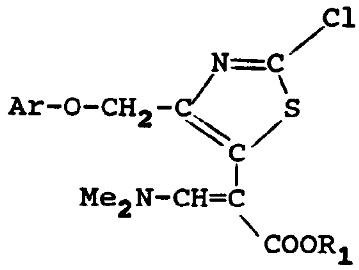

(XVI)$_2$,

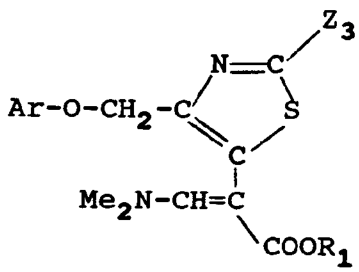

(XVI)$_3$ et

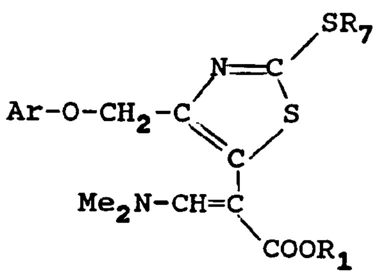

(XVI)$_4$

produits de formules (XVI)$_1$, (XVI)$_2$, (XVI)$_3$ et (XVI)$_4$ que l'on peut représenter sous la seule formule (XVI) :

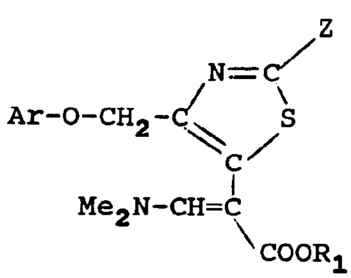

(XVI)

dans laquelle Z, Ar et $R_1$ a la signification ci-dessus, et produit de formule (XVI) qui, par hydrolyse est transformé

en produit de formule (XVII) :

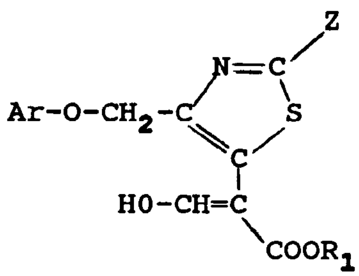

(XVII)

produit de formule (XVII) que l'on éthérifie en produit de formule (I).

**2)** Procédé selon la revendication 1, caractérisé en ce que l'on utilise au départ un produit de formule (II) dans laquelle $R_1$ représente un radical méthyle et en ce que l'éthérification du produit de formule (XVII) est effectuée au moyen d'un halogénure de méthyle.

**3)** Procédé selon la revendication 1 ou 2, caractérisé en ce que soit l'on traite un produit de formule (XI) par un agent de chloration, soit l'on traite un produit de formule (VII) par un réactif de formule (VIII) ou (XII) choisi de manière telle que l'on prépare un produit de formle ($XVI_1$) ou ($XVI_3$) dans lequel Z1 ou $Z_3$ représente un atome d'hydrogène, un radical méthyle, éthyle, propyle, isopropyle, méthoxy ou éthoxy, soit l'on traite un produit de formule (XII) par un réactif de formule (XV) dans laquelle R7 représente un radical méthyle.